# FASCICULE DE BREVET EUROPEEN

(11) **EP 2 237 733 B1**
(45) Date de publication et mention de la délivrance du brevet: **25.01.2012**
(21) Numéro de dépôt: 09706903.3
(22) Date de dépôt: 31.01.2009
(51) Int. Cl.: A61B 19/00

(54) **MANIPULATEUR A DECOUPLAGE DES MOUVEMENTS, ET APPLICATION AUX INSTRUMENTS POUR CHIRURGIE MINI INVASIVE**
MANIPULATOR MIT ENTKOPPELTEN BEWEGUNGEN UND SEINE ANWENDUNG IN INSTRUMENTEN FÜR MINIMAL INVASIVE CHIRURGIE
MANIPULATOR WITH DECOUPLED MOVEMENTS, AND APPLICATION TO INSTRUMENTS FOR MINIMALLY INVASIVE SURGERY

(30) Priorité: 31.01.2008 FR 0850612
(43) Date de publication de la demande: 13.10.2010
(73) Titulaire: Dexterite Surgical, 74000 Annecy (FR)
(72) Inventeur: BARRIER, Pascal, F-74000 Annecy (FR); OLLAGNIER, Jérémy, F-74940 Annecy Le Vieux (FR); GAUTIER, Gérard, F-74960 Cran Gevrier (FR)
(74) Mandataire: Poncet, Jean-François
(86) Numéro de dépôt international: PCT/IB2009/050390
(87) Numéro de publication internationale: WO 2009/095893

(56) Documents cités:
- WO-A-2005/046500
- WO-A-2006/005061
- FR-A- 2 876 271
- US-A1- 2003 033 024
- US-A1- 2003 109 957
- US-A1- 2006 020 287

## Description

### DOMAINE TECHNIQUE DE L'INVENTION

La présente invention concerne les dispositifs de guidage et de manipulation permettant de commander, depuis l'extérieur d'une zone d'intervention, les mouvements d'un instrument de manipulation situé à l'intérieur de la zone d'intervention.

En particulier, l'invention concerne un tel dispositif de guidage et de manipulation permettant de commander un instrument chirurgical dans les applications de chirurgie mini invasive pratiquées sous endoscopie.

Au cours des années récentes, la technique de chirurgie mini invasive sous endoscopie s'est développée considérablement.

On a pour cela conçu essentiellement deux types de dispositifs de guidage et de manipulation d'instrument chirurgical.

Selon le premier type de dispositif de guidage et de manipulation, décrit par exemple dans les documents FR 2 713 129 A ou FR 2 876 271 A, l'instrument chirurgical est placé en bout d'un manipulateur portable ayant essentiellement un bras principal à extrémité proximale et extrémité distale. L'extrémité proximale du bras porte une structure proximale de manipulation apte à générer des instructions de mouvement en fonction de sollicitations d'une main d'un opérateur. Une structure distale commandée est portée par l'extrémité distale du bras principal et est mobile par rapport à ladite extrémité distale de bras principal selon au moins deux degrés de liberté de pivotement transversal et éventuellement un degré de liberté de rotation axiale. Les mouvements appliqués par l'opérateur à la structure proximale de manipulation sont transmis, par des moyens de transmission dans le bras principal, et ces mouvements sont reproduits par la structure distale commandée.

Lors d'une opération chirurgicale, le bras principal traverse la peau d'un patient, la structure proximale de manipulation restant à l'extérieur du corps du patient, la structure distale commandée étant alors à l'intérieur du corps du patient pour réaliser le geste chirurgical.

Dans le dispositif du document FR 2 713 129 A, la structure proximale de manipulation comprend des zones de contact pour recevoir l'appui de deux doigts d'un opérateur, les zones de contact étant disposées à l'extrémité d'un levier articulé sur l'extrémité proximale du bras principal par une articulation à rotule. La structure distale commandée est disposée à l'extrémité d'un levier distal lui-même articulé à l'extrémité distale du bras principal par une seconde articulation à rotule. Les deux rotules sont reliées mécaniquement l'une à l'autre pour réaliser une reproduction de la rotation de la rotule proximale par la rotule distale.

Un premier inconvénient de ce dispositif est la difficulté de réaliser un geste chirurgical précis. On constate en effet une difficulté d'appliquer à la structure proximale de manipulation des sollicitations permettant de reproduire de façon précise des mouvements de rotation pure ou de translation pure de la structure distale commandée.

Un second inconvénient de ce dispositif est la relative faiblesse du débattement angulaire possible dans les mouvements de pivotement d'axe transversal de la structure distale commandée.

En ce qui concerne le dispositif décrit dans le document FR 2 876 271 A, les mêmes inconvénients sont à nouveau présents.

Ce document décrit en outre un mode de réalisation dans lequel la structure proximale de manipulation est déportée à l'écart du bras principal, et la transmission des mouvements entre la structure proximale de manipulation et la structure distale commandée se fait par l'intermédiaire de moyens moteurs alimentés par une source extérieure d'énergie.

Selon le deuxième type de dispositif de guidage et de manipulation pour instrument chirurgical tel que décrit par exemple dans le document US 5,797,900 A, l'instrument chirurgical est actionné par un robot télécommandé. Un bras maître, actionnable par un opérateur, est complètement séparé d'un bras esclave qui porte l'instrument chirurgical. Habituellement, le bras maître et le bras esclave sont des bras articulés ayant au moins six degrés de liberté de déplacement, le bras maître est muni de capteurs détectant les sollicitations qui lui sont appliquées, et le bras esclave est sollicité mécaniquement par des actionneurs motorisés qui répondent aux signaux émis par les capteurs du bras maître. Ainsi, la liaison entre le bras maître et le bras esclave est assurée par des signaux électriques, et le robot est généralement placé à distance du bras maître.

Un autre exemple de manipulateur à télécommande selon ce deuxième type de dispositif de guidage et manipulation est décrit dans le document US 2003/0109957 A1. Ce document décrit de manière plus détaillée une structure proximale de manipulation sous forme d'une poignée reliée à un support fixe par cinq articulations successives munies chacune de capteurs de déplacement pour commander à distance un bras esclave portant un instrument chirurgical. En partant du support fixe, une rotation d'épaule maître commande un pivotement latéral du bras esclave autour du trocart dans un premier plan longitudinal ; une rotation de coude maître commande un pivotement latéral du bras esclave autour du trocart dans un second plan longitudinal ; une translation axiale d'avant-bras maître commande une translation axiale du bras esclave dans le trocart ; une rotation axiale de poignet maître commande une rotation axiale du bras esclave dans le trocart ; enfin, une rotation transversale de la poignée commande encore une rotation du bras esclave. Les capteurs de déplacement sont tous, à l'exception du capteur de rotation transversale de poignée, situés dans des zones de liaison qui sont nettement à l'écart de la poignée. Ce dispositif à articulations successives n'est pas applicable à un manipulateur portable car le bras esclave doit alors être tenu mécaniquement par la structure proximale de manipulation, et les articulations ne permettent pas cette tenue. En outre, dans ce document, l'instrument chirurgical n'est pas mobile en orientation et en rotation par rapport au bras esclave.

Du fait de leur complexité, de tels manipulateurs à télécommande sont volumineux, onéreux, et nécessitent des coûts de maintenance élevés. L'opérateur actionne le bras maître en une position éloignée du patient, de sorte que l'opérateur ne peut pas agir pour administrer un traitement médical rapide au patient en cas d'urgence. Et l'encombrement du système nécessite un temps de préparation long et une réorganisation complète de la salle d'opération et des méthodes de travail.

On notera aussi que ces documents décrivent une structure distale commandée dans laquelle l'outil chirurgical ne peut pas exécuter un mouvement de rotation propre axiale autour de son axe, mouvement qui est parfois nécessaire dans les actes chirurgicaux. Les actes chirurgicaux nécessitent alors l'intervention de plusieurs mouvements selon des axes différents, ces mouvements devant être simultanés et synchronisés, ce qui nécessite des moyens de calcul relativement complexes, et ce qui complique encore le dispositif. US 2003/0033024 décrit un manipulateur selon le préambule de la revendication 1 suivante.

### EXPOSE DE L'INVENTION

Le problème proposé par la présente invention est de concevoir un nouveau type de manipulateur qui permette d'augmenter la précision du geste chirurgical sans toutefois nécessiter un encombrement et des investissements importants.

En particulier, l'invention vise à réaliser une augmentation de précision du geste chirurgical sans recourir à un robot télécommandé.

L'invention résulte de l'observation selon laquelle les manipulateurs connus de type portable ne permettent pas de discriminer efficacement les différentes sollicitations de mouvement appliquées à la structure proximale de manipulation. En particulier, une sollicitation de type pivotement relatif d'axe transversal appliquée à la structure proximale de manipulation induit généralement non seulement un mouvement de pivotement relatif similaire d'axe transversal de la structure distale commandée vis-à-vis du bras principal, mais aussi un mouvement de pivotement global et de translation globale axiale du bras principal lui-même par rapport au corps du patient.

Pour éviter ces inconvénients, l'invention propose un manipulateur comprenant :
- un bras principal, ayant une extrémité proximale et une extrémité distale,
- une structure proximale de manipulation, portée par l'extrémité proximale du bras principal à laquelle elle se raccorde par une structure de liaison, ayant au moins deux zones de contact opposées conformées pour recevoir l'appui de deux parties opposées d'une main d'un opérateur, et comprenant des capteurs de sollicitation aptes à générer des instructions de mouvement en fonction de sollicitations de la main de l'opérateur,
- une structure distale commandée, portée par l'extrémité distale du bras principal, et mobile par rapport à ladite extrémité distale selon au moins deux degrés de liberté de pivotement relatif transversal et un degré de liberté de rotation propre axiale,
- des moyens moteurs, alimentés par une source d'énergie, et aptes à générer des mouvements en fonction des instructions de mouvement reçues de la structure proximale de manipulation,
- des moyens de transmission mécanique, logés dans le bras principal, couplés mécaniquement aux moyens moteurs et aptes à transmettre les mouvements des moyens moteurs à la structure distale commandée pour générer les mouvements de la structure distale commandée en fonction des instructions de mouvement de la structure proximale de manipulation,
   et dans lequel :
- la structure de liaison est disposée dans une zone intermédiaire entre les zones de contact opposées de la structure proximale de manipulation,
- des capteurs de sollicitation sont disposés dans ladite structure de liaison et aptes à générer les instructions de mouvement de la structure distale commandée par rapport au bras principal selon au moins deux degrés de liberté de mouvement, en fonction des sollicitations détectées dans ladite structure de liaison.

Par le fait que des capteurs de sollicitation sont disposés et agencés de façon à générer des instructions de mouvements de la structure distale commandée en fonction des sollicitations présentes et détectées dans une structure de liaison située entre les deux zones de contact opposées, on réalise une discrimination des sollicitations de mouvement possibles reçues par la structure proximale de manipulation selon les différents degrés de liberté du manipulateur.

Selon une première possibilité, la structure de liaison comprend une articulation, et les capteurs sont des capteurs de déplacement sensibles au déplacement relatif du bras principal et de la structure proximale de manipulation de part et d'autre de l'articulation.

Selon une seconde possibilité, la structure de liaison est une structure élastiquement déformable, et les capteurs sont des jauges de contrainte sensibles à la déformation de la structure de liaison.

Selon un premier mode de réalisation pratique :
- la structure proximale de manipulation est déportée radialement à l'écart de l'axe longitudinal du bras principal,
- les zones de contact opposées sont, en-position moyenne, alignées selon une direction faisant avec l'axe longitudinal du bras principal un angle d'environ 45°,
- les capteurs de sollicitation disposés dans la structure de liaison sont aptes à générer les instructions de mouvement de la structure distale commandée selon les deux degrés de liberté de pivotement relatif transversal et selon le degré de liberté de rotation propre axiale.

De la sorte, les sollicitations de pivotement relatif centré appliquées sur la structure proximale de manipulation n'ont que très peu d'effet sur les autres mouvements possibles du manipulateur, notamment les mouvements de translation globale et de pivotement global du bras principal de manipulateur. On assure ainsi une bonne discrimination des mouvements possibles du manipulateur selon ses différents degrés de liberté, tout en commandant tous les mouvements par la sollicitation des seules deux zones de contact opposées de la structure proximale de manipulation.

Selon un second mode de réalisation pratique :
- la structure de liaison est centrée sur l'axe longitudinal du bras principal,
- les capteurs de sollicitation disposés dans la structure de liaison sont aptes à générer les instructions de mouvement de la structure distale commandée selon les deux degrés de liberté de pivotement relatif transversal,
- les mouvements de rotation propre axiale de la structure distale commandée sont commandés par les instructions de mouvement générées par un capteur de sollicitation supplémentaire, porté par la structure proximale de manipulation, et actionnable par un doigt de la main d'utilisateur agissant sur la structure proximale de manipulation.

De la sorte, on améliore encore la discrimination des mouvements possibles du manipulateur selon ses différents degrés de liberté.

Le manipulateur défini ci-dessus est un élément portatif que l'opérateur peut tenir à la main lors d'une intervention chirurgicale. Avantageusement, un tel manipulateur peut comprendre aussi un élément d'appui intermédiaire du bras principal, dans lequel le bras principal peut coulisser axialement, et qui peut pivoter selon un mouvement de pivotement global sphérique. On facilite ainsi le guidage de la structure distale commandée, pour maîtriser son positionnement vis-à-vis d'une zone d'intervention.

De préférence, le bras principal peut en outre avoir, par rapport à l'élément d'appui intermédiaire, un mouvement de rotation globale axiale.

En pratique, un manipulateur tel que défini ci-dessus peut avoir une structure distale commandée comprenant un support distal articulé en bout du bras principal, ledit support distal pouvant osciller de part et d'autre de l'axe longitudinal du bras principal selon des mouvements de pivotement relatif à deux degrés de liberté à axes transversaux concourants. Le support distal articulé porte une broche rotative porte-outil, apte à tourner en rotation propre axiale dans le support distal. On donne ainsi à l'outil chirurgical placé sur le manipulateur tous les degrés de mouvement relatif souhaités.

Dans tous les cas, on peut avantageusement prévoir que les capteurs de sollicitation, les moyens moteurs et les moyens de transmission mécanique soient agencés de façon que :
- une sollicitation de pivotement relatif centré appliquée sur la structure proximale de manipulation produit un pivotement relatif similaire du support distal de la structure distale commandée,
- une sollicitation de rotation propre axiale appliquée sur la structure proximale de manipulation produit une rotation relative axiale similaire de la broche rotative porte-outil.

Pour réaliser certains gestes chirurgicaux, la structure distale commandée doit pouvoir reproduire aisément des mouvements de pivotement relatif de grande amplitude selon deux axes transversaux concourants. Ainsi, selon un second aspect, l'invention propose une nouvelle structure distale commandée permettant à la fois de reproduire fidèlement les mouvements de pivotement relatif et d'augmenter l'amplitude maximale de tels mouvements par rapport aux capacités proposées par les dispositifs connus, tout en permettant des mouvements de rotation propre axiale d'un outil porté par la structure distale commandées.

Pour réaliser cette grande amplitude des mouvements de pivotement relatif de la structure distale commandée vis-à-vis du bras principal, l'invention propose une telle structure distale commandée qui comprend :
- à l'extrémité distale du bras principal, un élément d'articulation femelle à cavité hémisphérique distale,
- un support distal articulé, sous forme d'un élément d'articulation mâle en forme de collerette hémisphérique creuse, ayant une surface externe hémisphérique, et ayant un évidement intérieur largement ouvert vers sa base,
- l'élément d'articulation mâle étant engagé dans la cavité hémisphérique distale de l'élément d'articulation femelle, avec son évidement intérieur orienté vers la cavité hémisphérique distale,
- une pluralité de fils de commande s'étendant dans le bras principal, couplés mécaniquement aux moyens moteurs, et engagés en périphérie de la surface externe hémisphérique de l'élément d'articulation mâle pour en commander le pivotement par traction sur les fils de commande,
- une broche rotative porte-outil, sous forme d'un arbre de sortie, monté à rotation dans un palier radial de l'élément d'articulation mâle, et portant un outil ou un porte-outil,
- un arbre d'entrée, monté à rotation dans un palier axial de l'élément d'articulation femelle, et engagé longitudinalement dans le bras principal,
- une transmission homocinétique qui relie l'arbre d'entrée à l'arbre de sortie en autorisant les mouvements de pivotement transversal de l'élément d'articulation mâle dans l'élément d'articulation femelle.

On réalise ainsi une structure distale commandée ayant elle-même deux degrés de liberté de pivotement relatif d'axe transversal à axes concourants, et donnant un degré de liberté de rotation propre axiale à un outil chirurgical qu'elle porte, ces mouvements de pivotement relatif et de rotation propre étant totalement indépendants les uns des autres, et les mouvements de pivotement relatif pouvant atteindre des amplitudes de l'ordre de 70° de part et d'autre de l'axe longitudinal du bras principal.

Une telle structure distale commandée peut être utilisée sur un manipulateur indépendamment de la présence ou de l'absence des moyens particuliers ci-dessus permettant de discriminer les mouvements.

Un souci permanent est de réaliser un manipulateur portable qui soit à la fois peu onéreux et aisément manipulable. Ainsi, selon un troisième aspect, l'invention vise en outre à réduire le poids et l'encombrement d'un tel manipulateur.

Pour cela, l'invention propose de concevoir un tel manipulateur dans lequel l'arbre principal est engagé à coulissement dans un passage d'un trocart chirurgical ou d'un bras support formant élément d'appui intermédiaire, le trocart chirurgical ou le bras support ayant des moyens de connexion à contacts glissants pour transmettre l'énergie électrique et les signaux de commande et de contrôle entre le bras principal et un ensemble externe d'alimentation et de traitement.

De la sorte, la source d'énergie et les moyens de calcul peuvent être déportés à l'écart du manipulateur lui-même, et n'affectent pas son poids et son encombrement au voisinage du champ opératoire.

Un tel trocart ou bras support à contacts glissants peut être utilisé indépendamment de la présence ou de l'absence des autres moyens ci-dessus de discrimination des mouvements ou d'augmentation des amplitudes de pivotements relatifs.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 illustre schématiquement un opérateur actionnant deux manipulateurs selon la présente invention pour réaliser une opération chirurgicale ;
- la figure 2 est une vue schématique de côté d'un manipulateur selon un mode de réalisation de la présente invention ;
- la figure 3 illustre le manipulateur de la figure 2 lors d'un mouvement de petit pivotement de la structure distale commandée autour de son axe transversal ;
- la figure 4 illustre le manipulateur de la figure 2 lors d'un mouvement de grand pivotement autour d'un axe transversal du bras principal, et lors d'une rotation axiale de la structure distale commandée ;
- les figures 5, 6 et 7 illustrent, en perspective, les mouvements du manipulateur lors d'une opération chirurgicale de suture pour passage d'une aiguille de suture dans un tissu à suturer ;
- la figure 8 est une vue en perspective plus détaillée d'un manipulateur selon le mode de réalisation de la figure 2 ;
- la figure 9 est une vue en perspective du manipulateur de la figure 8, dans une position différente d'orientation de la structure distale commandée ;
- les figures 10 et 11 sont des vues schématiques illustrant les mouvements de rotation et de pivotement dans les manipulateurs de l'art antérieur ;
- la figure 12 illustre schématiquement les mouvements de pivotement et de rotation d'un manipulateur selon un mode de réalisation préféré de la présente invention ;
- les figures 13 et 14 sont des vues de côté schématiques, illustrant deux modes de réalisation de capteurs de sollicitation pour la structure proximale de manipulation ;
- la figure 15 est une vue en perspective d'une structure distale commandée selon un mode de réalisation particulier de la présente invention ;
- la figure 16 est une coupe longitudinale de la structure distale commandée selon la figure 15 ;
- la figure 17 est une vue schématique en coupe longitudinale de la structure de la figure 16, en position centrée ;
- la figure 18 est une coupe longitudinale de la structure de la figure 16 en position de pivotement intermédiaire ;
- la figure 19 est une coupe longitudinale de la structure de la figure 16 en position de pivotement extrême ;
- la figure 20 est une vue schématique de côté d'un manipulateur selon l'invention avec trocart chirurgical ;
- la figure 21 illustre le manipulateur de la figure 20 en perspective à l'état désassemblé ;
- la figure 22 illustre le manipulateur de la figure 21 à l'état assemblé.

### DESCRIPTION DES MODES DE REALISATION PREFERES

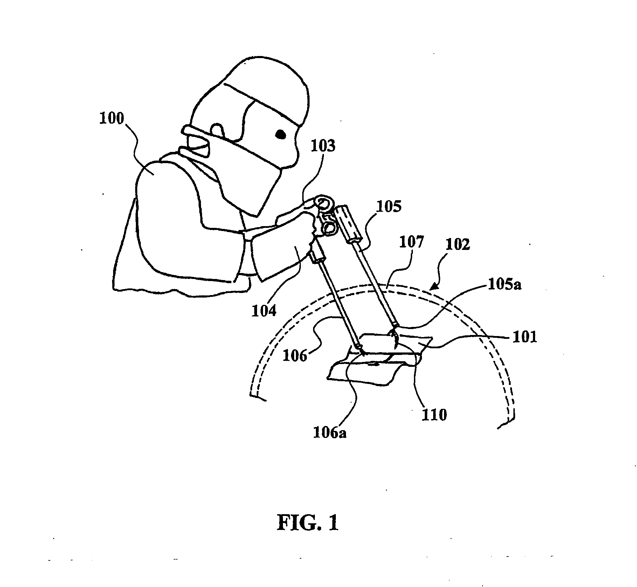

Comme illustré sur la figure 1, au cours d'une opération chirurgicale mini invasive, un opérateur 100 doit agir sur un tissu 101 situé à l'intérieur du corps 102 d'un patient. L'opérateur manipule, par ses mains 103 et 104, deux manipulateurs 105 et 106 permettant, par exemple, de réaliser une suture par passage d'une aiguille courbe 110 dans le tissu 101.

Chacun des deux manipulateurs 105 et 106 traverse la peau 107 du corps de patient 102, une portion distale de chaque manipulateur 105 et 106 se trouvant ainsi à l'intérieur du corps de patient 102, tandis qu'une portion proximale se trouve à l'extérieur pour être manipulée par l'opérateur 100.

L'invention vise à permettre à l'opérateur 100 d'exécuter des mouvements naturels et simples de ses mains 103 et 104 pour réaliser les mouvements nécessaires des outils chirurgicaux respectifs 105a et 106a disposés aux extrémités distales des manipulateurs respectifs 105 et 106.

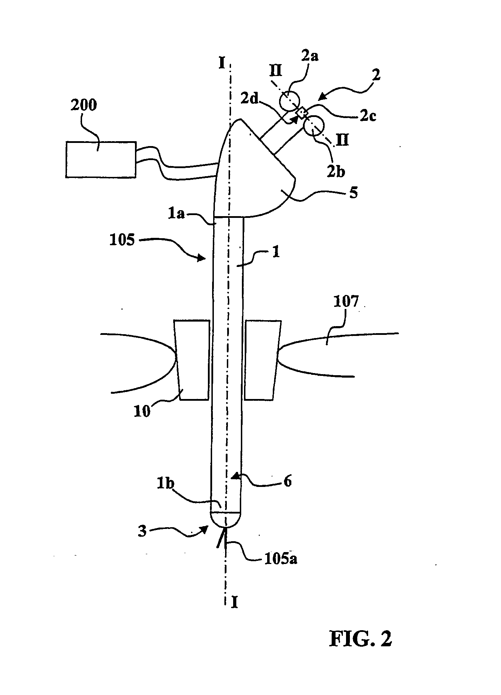

On considère maintenant la représentation schématique de la figure 2, qui illustre de façon plus détaillée la structure générale d'un manipulateur 105 selon un mode de réalisation de l'invention, en position d'opération chirurgicale par traversée de la peau 107 du patient.

Le manipulateur 105 comprend un bras principal 1 ayant une extrémité proximale la et une extrémité distale 1b, une structure proximale de manipulation 2, une structure distale commandée 3, et un trocart chirurgical 10.

Le trocart chirurgical 10 est engagé dans une perforation de la peau 107 du patient, et forme un canal dans lequel le bras principal 1 peut coulisser axialement pour un mouvement de translation globale, et le bras principal 1 peut éventuellement tourner en rotation globale axiale autour de son axe longitudinal I-I.

Le trocart chirurgical 10 peut lui-même pivoter de part et d'autre de l'axe de la perforation de la peau 107 du patient, pour autoriser des mouvements de pivotement global du bras principal 1 autour du point de pivotement constitué par la perforation de la peau 107 du patient.

La structure proximale de manipulation 2 comprend au moins deux zones de contact opposées 2a et 2b, conformées pour recevoir l'appui de deux parties opposées d'une main de l'opérateur. Des capteurs de sollicitation 2c sont disposés de façon à générer des instructions de mouvement en fonction des sollicitations présentes dans une structure de liaison 2d qui relie la structure proximale de manipulation 2 au bras principal 1 et qui est située entre les deux zones de contact opposées 2a et 2b.

La structure distale commandée 3 est portée par l'extrémité distale 1 b du bras principal 1, et est mobile par rapport à ladite extrémité distale 1b selon au moins deux degrés de liberté de pivotement relatif transversal et un degré de liberté de rotation propre axiale.

Des moyens moteurs 5, alimentés par une source d'énergie comprise dans des moyens d'alimentation et de commande 200, sont aptes à générer des mouvements en fonction des instructions de mouvement reçues de la structure proximale de manipulation 2. Dans le mode de réalisation illustré sur les figures, les moyens d'alimentation et de commande 200 sont déportés à l'écart du manipulateur 105, pour en réduire le poids et l'encombrement. Toutefois, on peut aussi concevoir de placer des moyens d'alimentation et de commande 200 dans le bras principal 1 lui-même, si ces moyens d'alimentation et de commande 200 sont suffisamment légers et peu encombrants.

Des moyens de transmission mécanique 6, logés dans le bras principal 1, permettent de transmettre à la structure distale commandée 3 les mouvements produits par les moyens moteurs 5, de façon à générer les mouvements de la structure distale commandée 3 en fonction des instructions de mouvement de la structure proximale.de manipulation 2.

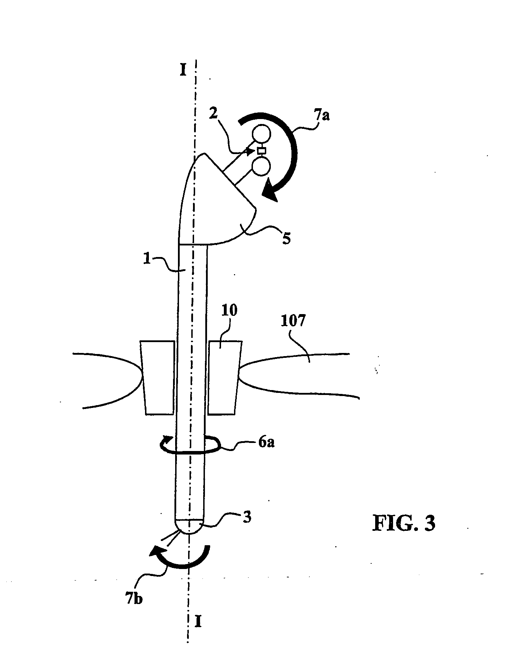

On considère, sur la figure 3, un premier type de mouvement de la structure distale commandée 3. Il s'agit d'un mouvement de pivotement relatif, ou de pivotement à faible rayon par rapport au bras principal 1, illustré par la flèche 7b, autour d'un axe transversal distal. Ce mouvement selon la flèche 7b est produit par une sollicitation de pivotement relatif sur elle-même de la structure proximale de manipulation 2, selon un pivotement relatif d'axe transversal proximal illustré par la flèche 7a. On notera que l'axe transversal distal autour duquel peut se produire la rotation 7b de la structure distale commandée 3 peut avoir toute orientation radiale autour de l'axe longitudinal I-I du bras principal 1. Pour cela, la structure proximale de manipulation 2 est pivotée autour d'un axe transversal proximal qui peut aussi avoir toute orientation radiale autour d'un axe longitudinal de la structure proximale de manipulation 2. En d'autres termes, la structure distale commandée 3 a deux degrés de liberté de pivotement transversal par rapport à l'axe longitudinal I-I du bras principal 1. La figure illustre aussi un mouvement de rotation globale axiale 6a du bras principal 1 autour de son axe longitudinal I-I.

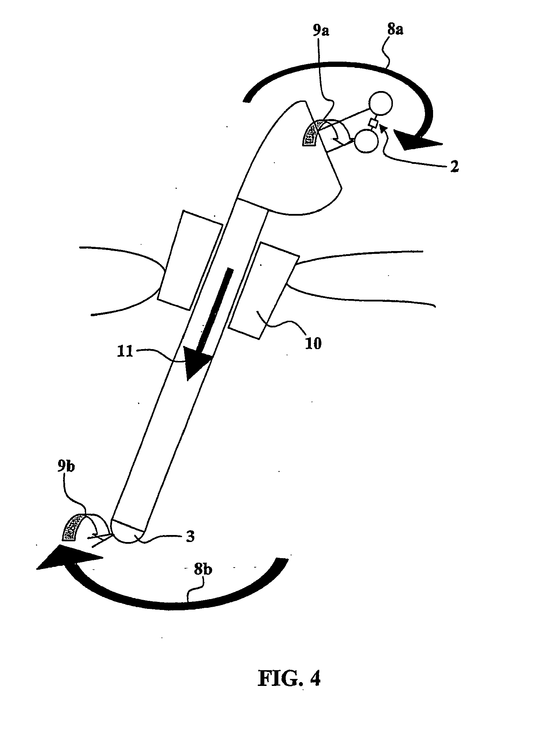

On considère maintenant la figure 4, qui illustre deux autres mouvements de la structure distale commandée 3 : d'une part un mouvement de pivotement global, ou pivotement à grand rayon, illustré par la flèche 8b, qui est une rotation du bras principal 1 autour d'un axe transversal intermédiaire dans la zone occupée par le trocart chirurgical 10, ce mouvement de pivotement global étant produit par une sollicitation 8a transversale appliquée par la main de l'opérateur sur la structure proximale de manipulation 2 ; d'autre part, un mouvement de rotation propre axiale illustré par la flèche 9b, produit par une sollicitation de rotation propre axiale similaire 9a appliquée par la main de l'opérateur sur la structure proximale de manipulation 2. La figure 4 illustre en outre un mouvement de translation globale axiale du bras principal 1 dans le trocart chirurgical 10, comme illustré par la flèche 11.

De tels mouvements sont nécessaires pour réaliser de façon simple et ergonomique les différents gestes chirurgicaux. En fonction du geste à réaliser, les mouvements peuvent différer en amplitude et être combinés les uns avec les autres.

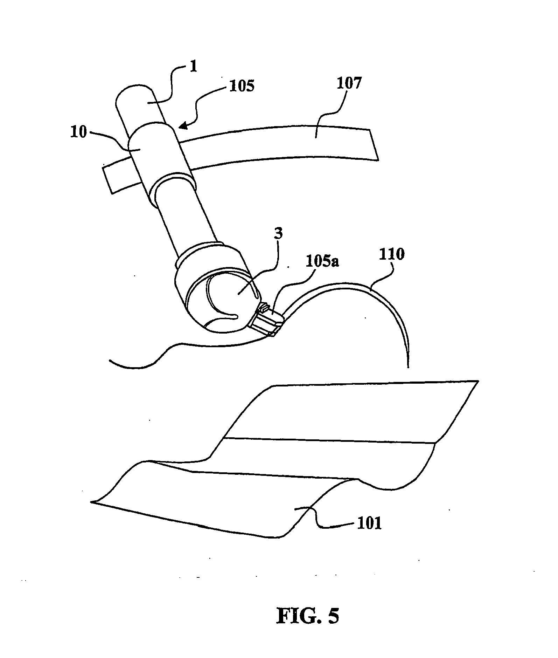

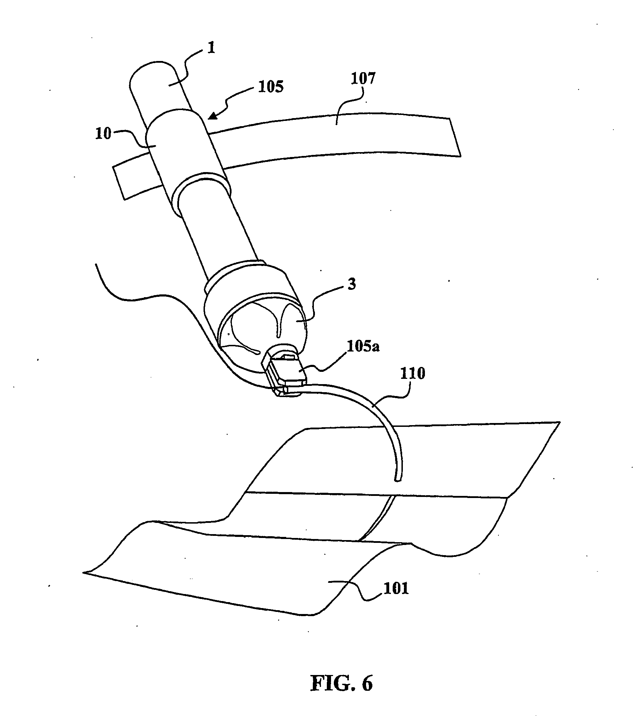

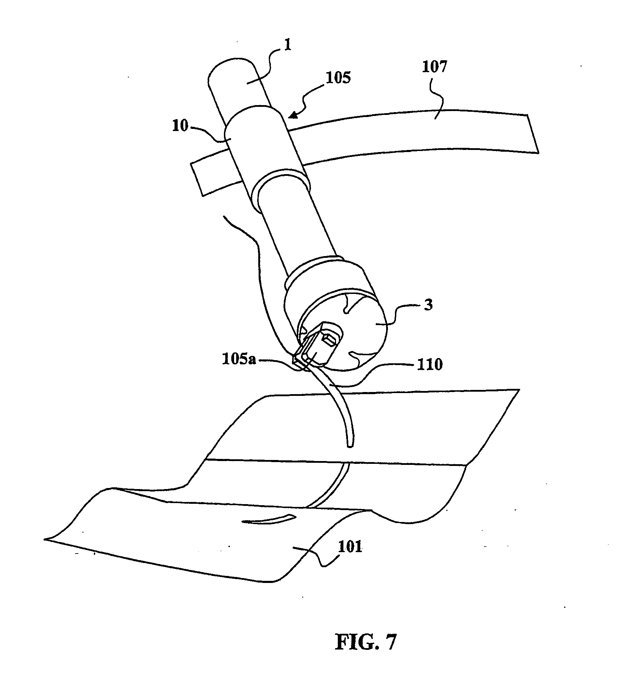

A titre d'exemple, on peut considérer les figures 5 à 7, qui illustrent les mouvements nécessaires pour un geste de passage d'une aiguille de suture 110 dans un tissu 101.

Dans ce cas, la structure distale commandée 3 porte un outil chirurgical 105a sous forme d'une pince avec laquelle l'opérateur vient pincer une extrémité proximale de l'aiguille de suture 110, courbe. En appliquant à la structure distale commandée 3 les mouvements de pivotement relatif, de rotation propre relative, de pivotement global, de rotation globale et de translation globale appropriés, l'opérateur peut ainsi passer l'aiguille de suture 110 courbe dans le tissu 101 comme illustré sur les figures. On comprend que le geste chirurgical est relativement complexe, et qu'il y a tout intérêt à simplifier les mouvements que l'opérateur doit appliquer à la structure proximale de manipulation 2 pour réaliser ce geste.

La précision du geste opératoire nécessite en particulier que les sollicitations appliquées par l'opérateur sur la structure proximale de manipulation 2 soient le plus naturelles possible, afin que tout se passe pour l'opérateur comme si il tenait à la main l'aiguille de suture 110 courbe.

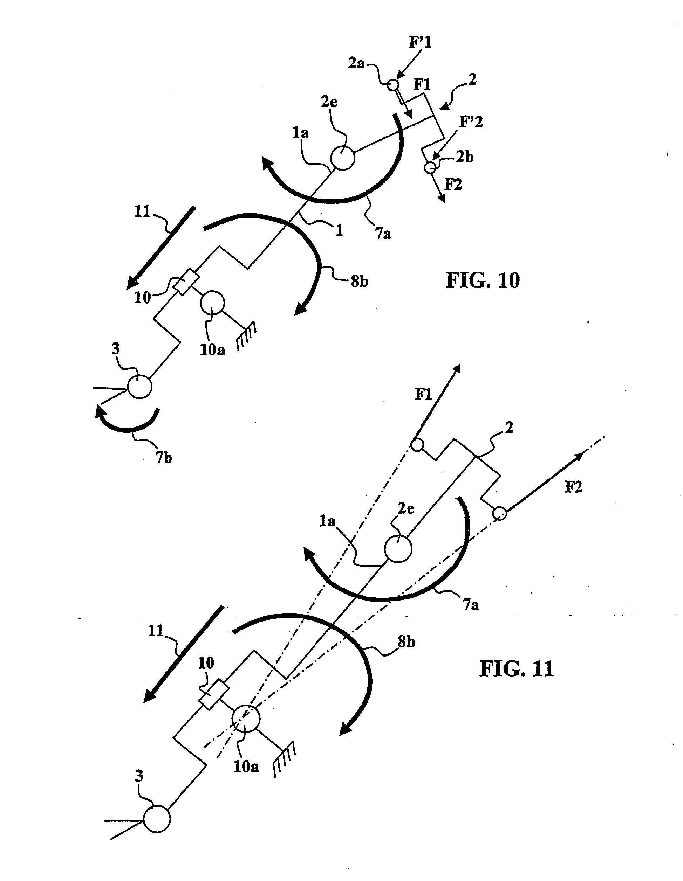

Pour comprendre cette difficulté, et l'intérêt de l'invention, on considère maintenant les figures 10 à 12.

La figure 10 illustre une structure connue de manipulateur, telle que celle décrite par exemple sur les documents FR 2 713 129 A ou FR 2 876 271 A.

Dans ce cas, on retrouve une structure proximale de manipulation 2, un bras principal 1, un trocart chirurgical 10 et une structure distale commandée 3.

La structure proximale de manipulation 2 comporte deux zones de contact 2a et 2b déportées, et est articulée à l'extrémité proximale 1a du bras principal 1 selon une zone de liaison de type articulation 2e, pouvant ainsi pivoter autour de l'articulation 2e. Le bras principal 1 peut pivoter avec le trocart chirurgical 10 par rapport à la peau de l'utilisateur selon un axe de pivotement 10a transversal.

Pour produire un mouvement de pivotement relatif 7b de la structure distale commandée 3, l'opérateur doit appliquer sur la structure proximale de manipulation 2 des forces F1 et F2 transversales tendant à faire pivoter la structure proximale de manipulation 2 autour de l'articulation 2e. Du fait que, dans les documents antérieurs, la structure proximale de manipulation 2 est déportée à l'écart de l'articulation 2e, les forces F1 et F2 transversales sont de même direction, et induisent simultanément un couple de pivotement global du bras principal 1 autour de l'axe 10a. Ainsi, le mouvement naturel de l'opérateur pour faire pivoter la structure proximale de manipulation 2 et produire un mouvement de pivotement relatif 7b induit simultanément un mouvement de pivotement global 8b, et éventuellement un mouvement de translation globale 11.

Si l'opérateur veut obtenir un mouvement de pivotement relatif 7b pur, il doit alors compenser le mouvement de translation 11 et le mouvement de pivotement global 8b, ce qui n'est pas naturel et nécessite un apprentissage et un contrôle visuel permanent du champ opératoire.

Pour obtenir une translation globale pure selon la flèche 11, l'action intuitive de l'opérateur est de tirer ou pousser le manipulateur dans l'axe du bras principal 1. En appliquant de telles forces F'1 et F'2 parallèles et égales sur les zones de contact opposées 2a et 2b, cela produit simultanément un pivotement relatif de la structure proximale de manipulation 2 autour de l'articulation 2e. L'opérateur doit alors corriger cela en modifiant la direction des forces appliquées ou en appliquant simultanément un couple de compensation sur la structure proximale de manipulation 2, ce qui n'est pas naturel.

Dans le cas de la figure 11, la translation globale pure selon la flèche 11 peut être obtenue un peu plus aisément avec les dispositifs connus par des forces F1 et F2, mais cela n'est qu'un cas particulier, dans lequel la structure proximale de manipulation 2 est dans l'axe du bras principal 1.

Sur la figure 10, pour réaliser un mouvement de pivotement global selon la flèche 8b, l'opérateur doit appliquer sur les zones de contact 2a et 2b des forces transversales F1 et F2 vis-à-vis de la direction du bras principal 1, pour obtenir son pivotement autour de l'axe transversal 10a. Du fait du décalage de la structure proximale de manipulation 2 à l'écart de l'articulation 2e, cette sollicitation produit simultanément un couple de pivotement de la structure proximale de manipulation 2 autour de l'articulation 2e, comme illustré par la flèche 7a. Cette sollicitation produit simultanément un pivotement relatif 7b de la structure distale commandée 3, ce qui n'est pas souhaité. L'opérateur doit donc compenser ce mouvement parasite par l'application simultanée d'un couple de compensation sur la structure proximale de manipulation 2.

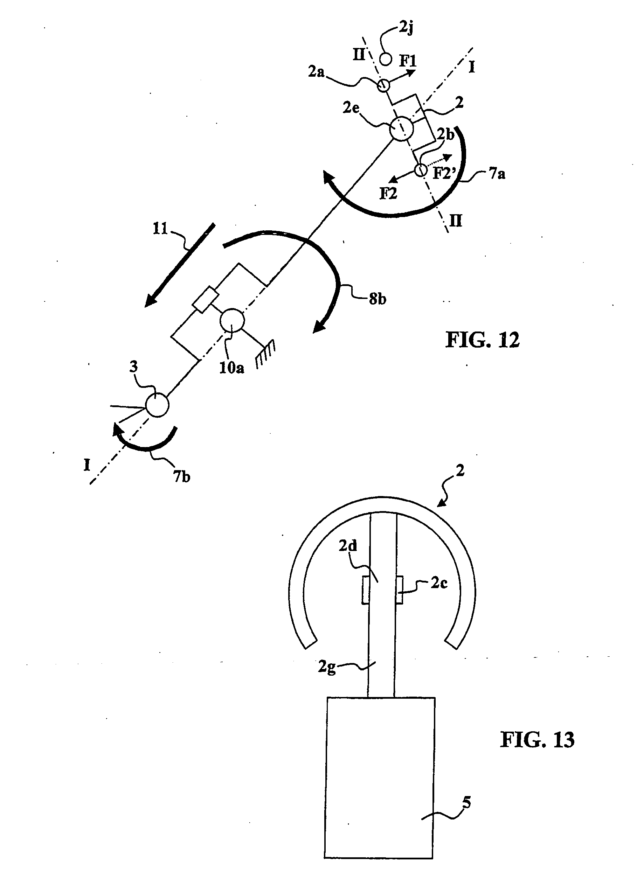

Considérons maintenant la figure 12, qui illustre schématiquement les mouvements dans un manipulateur selon un mode de réalisation préféré de la présente invention. Dans ce cas, l'articulation 2e est située sur l'axe longitudinal I-I du bras principal 1.

Dans tous les cas, l'application de deux forces égales F1 et F2' et de même sens sur les zones de contact opposées 2a et 2b n'induit aucun couple de rotation de la structure proximale de manipulation 2 autour de son articulation 2e, du fait que les deux zones de contact opposées sont alignées avec l'articulation 2e. Ainsi, des sollicitations pour pivotement global autour de l'axe 10a, ou des sollicitations pour translation globale 11, n'induisent aucune composante de pivotement relatif 7a de la structure proximale de manipulation 2 et de pivotement relatif 7b consécutif de la structure distale commandée 3.

De même, un pivotement relatif autour de l'articulation 2e selon la flèche 7a est obtenu par l'application de deux forces F1 et F2 égales et de sens opposé, ce qui n'entraîne aucune sollicitation de translation globale 11 ni aucune sollicitation de pivotement global 7a autour de l'axe 10a.

Pour discriminer efficacement le mouvement de rotation axiale propre de la structure distale commandée 3, on prévoit dans ce cas que ce mouvement de rotation soit piloté par un capteur de sollicitation supplémentaire 2j, porté par la structure proximale de manipulation 2, et actionnable par un doigt libre de la main d'utilisateur lorsqu'elle agit sur la structure proximale de manipulation 2. Un tel capteur 2j peut être un curseur, un bouton, une molette, par exemple.

L'invention réalise ainsi une dissociation parfaite des mouvements, l'opérateur pouvant appliquer les sollicitations intuitives qui normalement conduisent aux mouvements recherchés lorsqu'il tient un instrument chirurgical à la main.

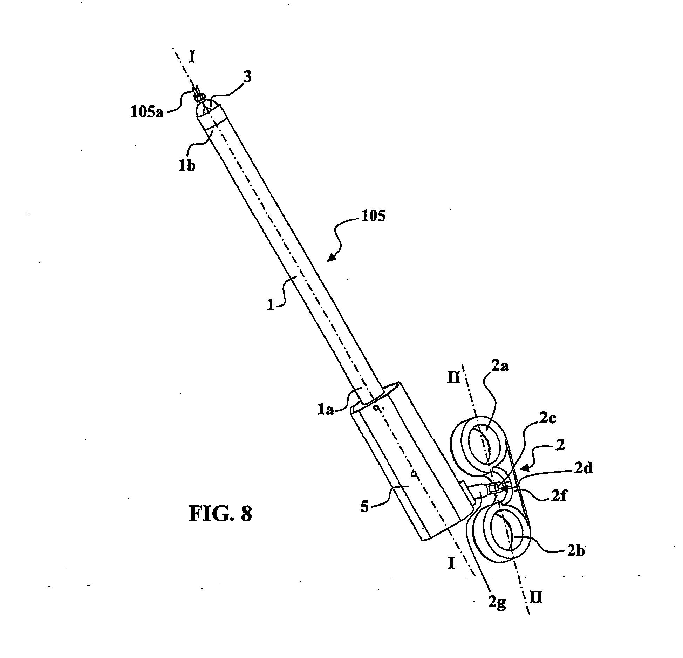

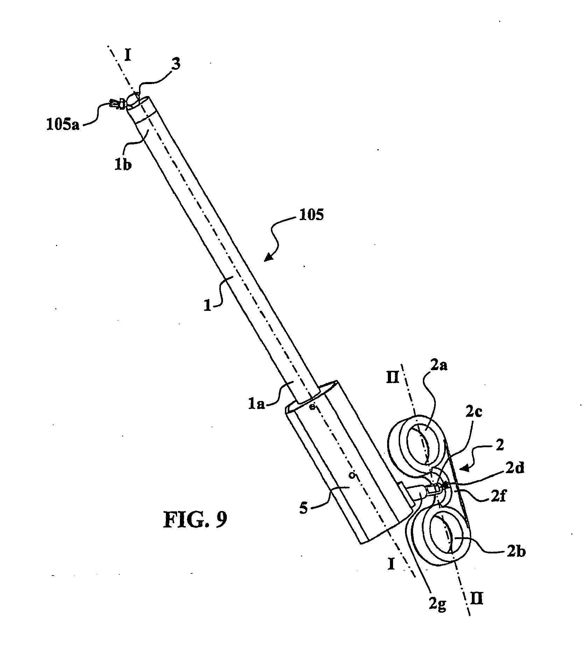

On considère maintenant les figures 8 et 9 qui illustrent en perspective une structure de manipulateur selon l'invention dans un autre mode de réalisation.

On retrouve sur ces figures les parties essentielles du manipulateur des figures 2 à 4, et ces parties essentielles sont repérées par les mêmes références numériques. Le trocart n'est pas représenté.

On distingue, à l'extrémité distale du manipulateur 105, l'instrument chirurgical 105a sous forme d'une pince, tenue par la structure distale commandée 3. Sur la figure 8, l'instrument chirurgical 105a est dans l'axe du bras principal 1.

Sur la figure 9, l'instrument chirurgical 105a est pivoté, selon un mouvement de pivotement relatif par rapport au bras principal 1.

Les figures illustrent un mode de réalisation avantageux d'une structure proximale de manipulation 2.

Dans ce cas, la structure proximale de manipulation 2 comprend deux anneaux coplanaires constituant les zones de contact opposées 2a et 2b, conformés de façon que l'opérateur puisse passer un doigt de main dans chacun des deux anneaux 2a et 2b.

De préférence, l'utilisateur passera un pouce dans l'un des anneaux, et un doigt de la même main dans l'autre anneau.

Les anneaux 2a et 2b sont reliés par une traverse 2f elle-même reliée aux moyens moteurs 5 et au bras principal 1 par un bras de liaison 2g. La traverse 2f et le bras de liaison 2g forment une structure qui porte les anneaux ou zones de contact opposées 2a et 2b. Une zone intermédiaire du bras de liaison 2g constitue la structure de liaison 2d centrale dans laquelle on souhaite capter les sollicitations appliquées par l'opérateur. Le bras de liaison 2g est orienté selon une direction radiale, ou au moins fortement inclinée par rapport à l'axe longitudinal I-I du bras principal 1, de sorte que les anneaux 2a et 2b sont déportés radialement à l'écart du bras principal 1, et les anneaux 2a et 2b sont alignés selon une direction II-II qui fait avec l'axe I-I un angle d'environ 45°.

Selon une première possibilité, la structure de liaison 2d du bras 2g est une zone articulée, et les capteurs 2c sont alors des capteurs de déplacement, par exemple, des encodeurs ou des potentiomètres, aptes à évaluer le pivotement relatif des deux tronçons successifs du bras de liaison 2g l'un par rapport à l'autre, pour produire les instructions de mouvement. Ainsi, dans cette réalisation, les capteurs 2c sont dans la structure de liaison 2d elle-même.

Selon une autre possibilité, la structure de liaison 2d du bras de liaison 2g est une structure élastiquement déformable, les capteurs étant alors des jauges de contrainte 2c sensibles à la déformation de cette structure de liaison 2d.

On peut aussi concevoir de placer les jauges de contrainte à l'écart du centre de la structure de liaison 2d, en corrigeant par calcul les contraintes mesurées par les capteurs 2c dans une zone déportée pour évaluer les contraintes présentes dans la zone intermédiaire 2d.

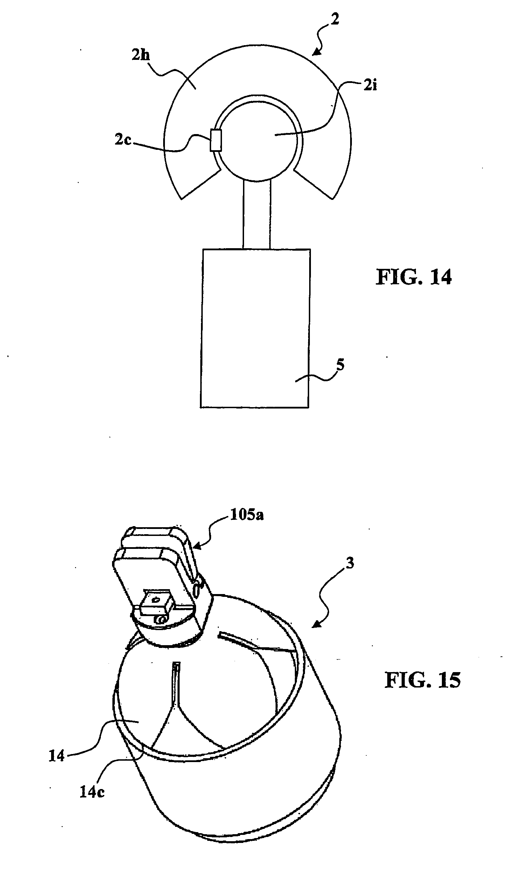

On considère maintenant les figures 13 et 14 qui illustrent deux autres modes de réalisation de la structure proximale de manipulation 2.

Dans ces deux cas, la structure proximale de manipulation 2 comprend une surface externe convexe, par exemple sphérique, dont deux portions diamétralement opposées constituent les zones de contact opposées, l'opérateur pouvant appliquer sa main sur la surface externe de la structure.

Sur la figure 13, la structure convexe est reliée aux moyens moteurs 5 par un bras de liaison 2g non articulé, dont la zone intermédiaire 2d est élastiquement flexible et comporte des jauges de contrainte 2c.

Sur la figure 14, une sphère périphérique 2h peut pivoter autour d'une rotule centrale 2i, et des capteurs de mouvements 2c déterminent les mouvements de la sphère périphérique 2h autour de la rotule centrale 2i pour générer les instructions de mouvement.

On considère maintenant les figures 15 à 19, qui illustrent plus spécialement un mode de réalisation avantageux de la structure distale commandée 3.

Le but de cette structure particulière est de permettre des mouvements de pivotement relatif de grande amplitude, pouvant atteindre environ 70° d'inclinaison de part et d'autre de l'axe longitudinal I-I du bras principal 1.

Un autre but de cette structure est de permettre ce pivotement par rotation centrée, donnant deux degrés de liberté de pivotement selon deux axes transversaux concourants. Autrement dit, la structure distale commandée 3 permet d'incliner de façon régulière et précise un outil chirurgical 105a dans toutes les directions tout autour de l'axe longitudinal I-I du bras principal 1.

Un autre but de cette structure est de permettre simultanément une rotation propre axiale de l'outil chirurgical 105a autour de son axe longitudinal, indépendamment des mouvements de pivotement relatif de part et d'autre de l'axe longitudinal I-I du bras principal 1.

On permet ainsi trois degrés de liberté indépendants les uns des autres, à savoir deux degrés de liberté de pivotement relatif selon des axes transversaux concourants, et un degré de liberté de rotation propre axiale de l'outil chirurgical 105a sur lui-même.

En outre, la structure distale commandée 3 permet une bonne proportionnalité des mouvements selon chacun des trois degrés de liberté, par rapport aux sollicitations correspondantes appliquées sur la structure proximale de manipulation 2.

Dans ce mode de réalisation représenté sur les figures 15 à 19, la structure distale commandée 3 comprend, à l'extrémité distale 1 b du bras principal 1, un élément d'articulation femelle 12 à cavité hémisphérique distale 13, et un élément d'articulation mâle 14 en forme de calotte hémisphérique creuse, ayant une surface externe 14a hémisphérique et ayant un évidement intérieur 14b largement ouvert vers sa base 14c.

L'élément d'articulation mâle 14 est engagé dans la cavité hémisphérique distale 13 de l'élément d'articulation femelle 12, avec son évidement intérieur 14b orienté vers la cavité hémisphérique distale 13.

Un arbre de sortie 15 est monté à rotation dans un palier radial 16 de l'élément d'articulation mâle 14, et porte l'outil 1 05a ou un porte-outil 105b.

Un arbre d'entrée 17 est monté à rotation dans un palier axial 18 de l'élément d'articulation femelle 12, et s'engage longitudinalement dans le bras principal 1.

Une transmission homocinétique 19 relie l'arbre d'entrée 17 à l'arbre de sortie 15 pour transmettre les mouvements de rotation axiale, tout en autorisant les mouvements de pivotement transversal de l'élément d'articulation mâle 14 dans l'élément d'articulation femelle 12.

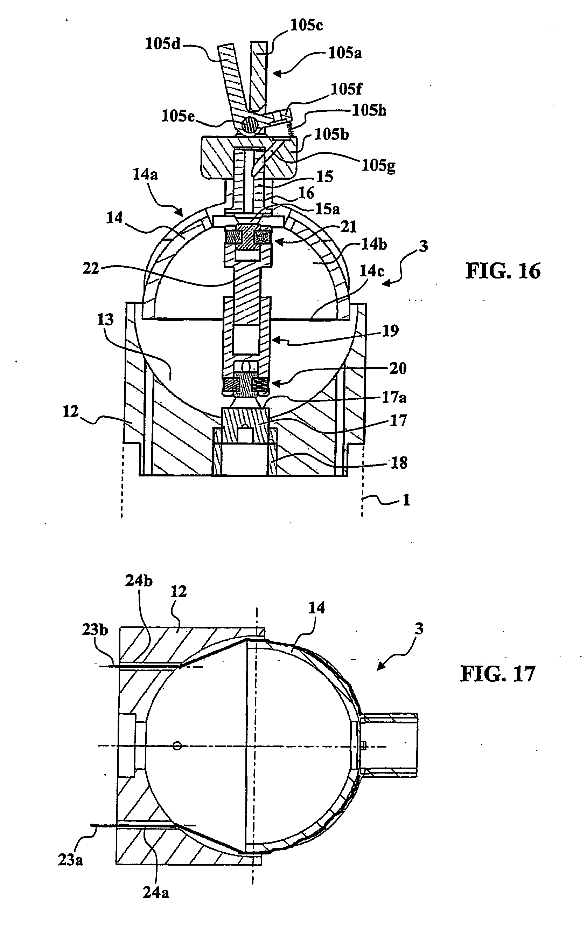

Dans la réalisation illustrée sur la figure 16, la transmission homocinétique 19 comprend un joint de cardan proximal 20 monté à l'extrémité intérieure 17a de l'arbre d'entrée 17, un joint de cardan distal 21 monté à l'extrémité intérieure 15a de l'arbre de sortie 15, et un arbre de transmission télescopique 22 qui relie le joint de cardan distal 21 au joint de cardan proximal 20. Les joints de cardan 20 et 21 sont décalés angulairement de 90°, comme illustré sur la figure, et sont équidistants de l'axe central de l'articulation sphérique constituée par les éléments d'articulation femelle 12 et mâle 14.

En alternative, on peut remplacer les deux joints de cardan (20-21) par un joint tripode assurant également une transmission homocinétique.

Dans la réalisation illustrée sur cette même figure 16, on voit que l'outil chirurgical 105a est une pince à deux mors 105c et 105d, le mors 105d étant mobile autour d'un axe transversal 1 05e pour produire un effet de pincement.

Dans la réalisation illustrée, le mors mobile 105d peut être sollicité par un câble de traction, non représenté, accroché à un bras transversal 105f et traversant des passages tels que le passage 105g pour le conduire jusqu'aux moyens moteurs, avec un ressort de rappel 105h repoussant le mors mobile 105d en position écartée.

En alternative, on peut concevoir un outil 105a en alliage à mémoire de forme, pouvant prendre les deux positions serrée et ouverte en fonction d'une température déterminée par une source de chaleur pilotée par les moyens moteurs.

Comme on le voit sur les figures 17 à 19, une pluralité de fils de commande, tels que les fils 23a et 23b, s'étendent dans le bras principal 1 et sont engagés en périphérie de la surface externe hémisphérique 14a de l'élément d'articulation mâle 14, surface périphérique à laquelle ils sont fixés.

A leur autre extrémité, les fils de commande sont couplés mécaniquement aux moyens moteurs 5 (figure 8), qui sont eux-mêmes adaptés pour assurer la traction sélective des fils de commande 23a, 23b. Les moyens moteurs 5 sont alimentés par un dispositif de commande électronique, pouvant avantageusement comporter des moyens de filtrage ou de lissage pour produire des tractions régulières sur les fils de commande 23a et 23b et éviter ainsi de transmettre les tremblements éventuels de l'opérateur.

A leur entrée dans l'élément d'articulation femelle 12, les fils de commande tels que les fils 23a et 23b traversent des passages longitudinaux de guidage périphériques respectifs 24a et 24b, qui assurent leur guidage pour assurer de la même manière le guidage en rotation axiale de l'élément d'articulation mâle 14. Les passages de guidage 24a et 24b sont de préférence au plus près de la surface périphérique de l'élément d'articulation femelle 12. Sur l'élément d'articulation mâle 14, les fils de commande, tels que les fils 23a et 23b, s'engagent sur la surface sphérique de l'élément d'articulation mâle 14.

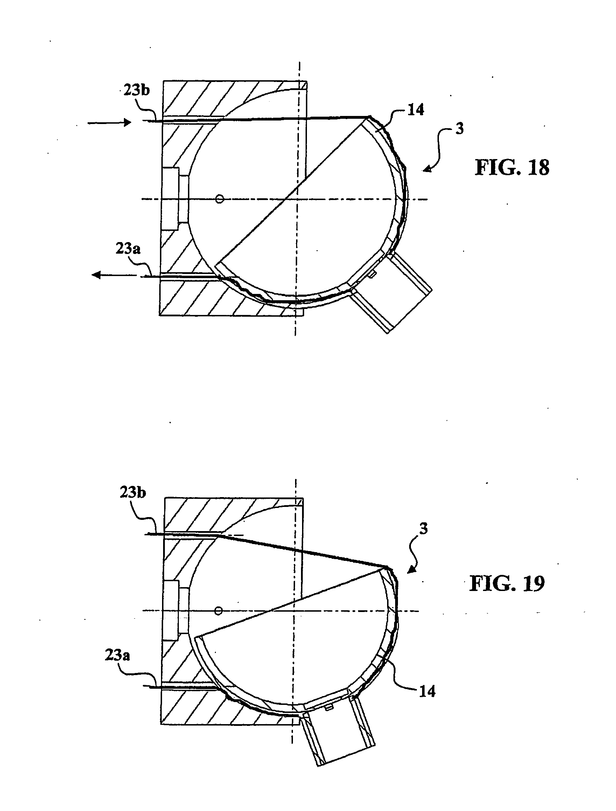

En pratique, dans les moyens moteur 5, les fils de commande 23a et 23b sont sollicités par des actionneurs linéaires pilotés par un dispositif de commande. Par exemple, les fils de commande diamétralement opposés 23a et 23b peuvent être couplés deux à deux, avec une poulie de retour proximale, et avec un actionneur commandant la translation simultanée des deux fils de commande 23a et 23b dans des sens opposés (figure 18).

Sur la figure 18, la translation simultanée en sens opposés des fils de commande 23a et 23b a produit un pivotement relatif d'environ 45 degrés de l'élément d'articulation mâle 14. Sur la figure 19, le pivotement relatif a été accentué, jusqu'à un maximum d'environ 70 degrés.

La zone distale du bras principal 1, comprenant la structure distale commandée 3, peut avantageusement être enveloppée d'une gaine souple, par exemple en polymère souple et fin, laissant dépasser l'outil chirurgical.

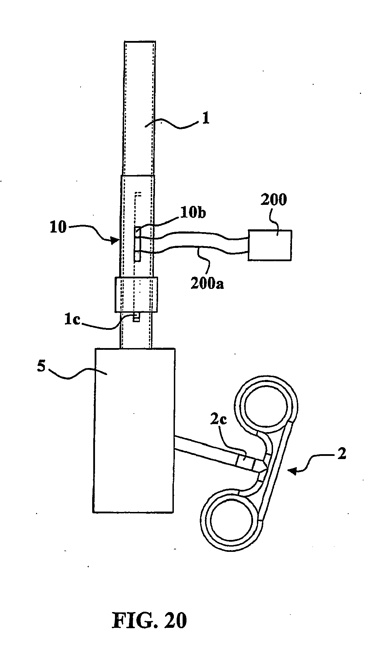

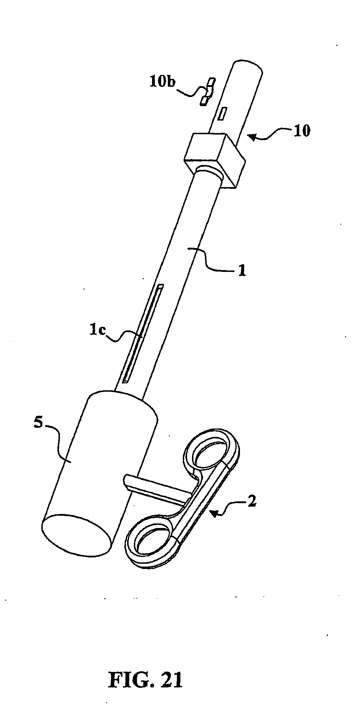

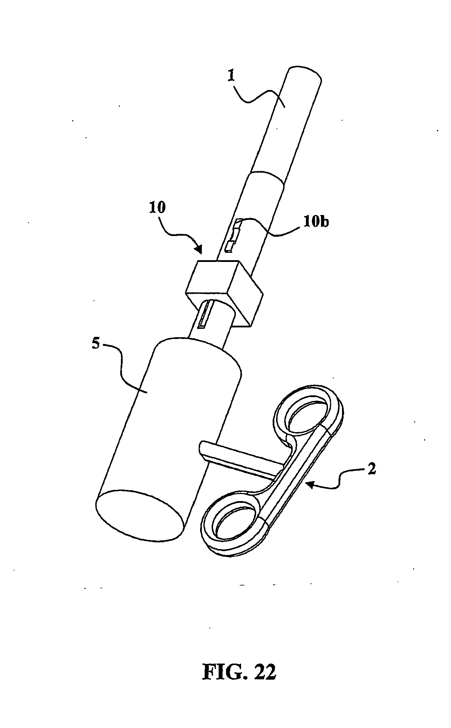

On considère maintenant les figures 20 à 22 qui illustrent une structure particulièrement avantageuse d'un manipulateur avec trocart chirurgical 10.

L'objet de cette structure est de réduire sensiblement le poids et l'encombrement du manipulateur lui-même, en déportant à l'écart les moyens d'alimentation de puissance et les moyens de traitement des signaux.

Pour cela, le trocart chirurgical 10 est un élément tubulaire comportant des moyens de contacts glissants 10b connectés électriquement à un moyen extérieur d'alimentation est de commande 200 comportant une alimentation électrique et des moyens de traitement de signaux.

Les conducteurs électriques 200a comprennent ainsi des conducteurs de puissance, et des conducteurs de signaux.

Les moyens de contacts glissants 10b sont connectés à une piste conductrice multiple 1c du bras principal 1, laquelle piste conductrice multiple étant connectée d'une part aux moyens moteurs 5 pour l'alimentation électrique des moyens moteurs 5, et d'autre part à des capteurs de sollicitation 2c de la structure proximale de manipulation 2.

La présente invention n'est pas limitée aux modes de réalisation qui ont été explicitement décrits, mais elle en inclut les diverses variantes et généralisations contenues dans le domaine des revendications ci-après.

## Revendications

1. Manipulateur, comprenant :
- un bras principal (1), ayant une extrémité proximale (1a) et une extrémité distale (1b),
- une structure proximale de manipulation (2), portée par l'extrémité proximale (1 a) du bras principal (1) à laquelle elle se raccorde par une structure de liaison (2e, 2f, 2g), ayant au moins deux zones de contact opposées (2a, 2b) conformées pour recevoir l'appui de deux parties opposées d'une main d'un opérateur (100), et comprenant des capteurs de sollicitation (2c) aptes à générer des instructions de mouvement en fonction de sollicitations de la main de l'opérateur (100),
- une structure distale commandée (3), portée par l'extrémité distale (1 b) du bras principal (1), et mobile par rapport à ladite extrémité distale (1 b) selon au moins deux degrés de liberté de pivotement relatif transversal (7b),
- des moyens moteurs (5), alimentés par une source d'énergie (200), et aptes à générer des mouvements en fonction des instructions de mouvement reçues de la structure proximale de manipulation (2),
- des moyens de transmission mécanique (6), logés dans le bras principal (1), couplés mécaniquement aux moyens moteurs (5) et aptes à transmettre les mouvements des moyens moteurs (5) à la structure distale commandée (3) pour générer les mouvements de la structure distale commandée (3) en fonction des instructions de mouvement de la structure proximale de manipulation (2), **caractérisé en ce que** :
- la structrure distale commandée (3) est mobile par rapport à la dite extrémité distale (1b) selon un degré de liberté de rotation propre axiale (9b);
- la structure de liaison (2e, 2f, 2g) est disposée dans une zone intermédiaire entre les zones de contact opposées (2a, 2b) de la structure proximale de manipulation (2),
- des capteurs de sollicitation (2c) sont disposés dans ladite structure de liaison (2e, 2f, 2g) et aptes à générer les instructions de mouvement de la structure distale commandée (3) par rapport au bras principal (1) selon au moins deux degrés de liberté de mouvement, en fonction des sollicitations détectées dans ladite structure de liaison (2e, 2f, 2g).

2. Manipulateur selon la revendication 1, **caractérisé en ce que** la structure de liaison (2e, 2f, 2g) comprend une articulation, et les capteurs (2c) sont des capteurs de déplacement sensibles au déplacement relatif du bras principal (1) et de la structure proximale de manipulation (2) de part et d'autre de l'articulation.

3. Manipulateur selon la revendication 1, **caractérisé en ce que** la structure de liaison (2e 2f, 2g) est une structure élastiquement déformable, et les capteurs sont des jauges de contrainte sensibles à la déformation de la structure de liaison (2e, 2f, 2g).

4. Manipulateur selon l'une quelconque des revendications 1 à 3, **caractérisé en ce que** :
- la structure proximale de manipulation (2) est déportée radialement à l'écart de l'axe longitudinal (I-I) du bras principal (1),
- les zones de contact opposées (2a, 2b) sont, en position moyenne, alignées selon une direction (II-II) faisant avec l'axe longitudinal (I-I) du bras principal (1) un angle d'environ 45°,
- les capteurs de sollicitation (2c) disposés dans la structure de liaison (2e, 2f, 2g) sont aptes à générer les instructions de mouvement de la structure distale commandée (3) selon les deux degrés de liberté de pivotement relatif transversal (7b) et selon le degré de liberté de rotation propre axiale (9b).

5. Manipulateur selon l'une quelconque des revendications 1 à 3, **caractérisé en ce que** :
- la structure de liaison (2e, 2f, 2g) est centrée sur l'axe longitudinal (I-I) du bras principal (1),
- les capteurs de sollicitation (2c) disposés dans la structure de liaison (2e, 2f, 2g) sont aptes à générer les instructions de mouvement de la structure distale commandée (3) selon les deux degrés de liberté de pivotement relatif transversal (7b),
- les mouvements de rotation propre axiale de la structure distale commandée (3) sont commandés par les instructions de mouvement générées par un capteur de sollicitation supplémentaire (2j), porté par la structure proximale de manipulation (2), et actionnable par un doigt de la main d'utilisateur agissant sur la structure proximale de manipulation (2).

6. Manipulateur selon l'une quelconque des revendications 1 à 5, **caractérisé en ce que** la structure proximale de manipulation (2) comprend une surface externe convexe dont deux portions diamétralement opposées constituent lesdites zones de contact opposées (2a, 2b).

7. Manipulateur selon l'une quelconque des revendications 1 à 5, **caractérisé en ce que** la structure proximale de manipulation (2) comprend deux anneaux coplanaires constituant lesdites zones de contact opposées (2a, 2b) et reliés par une traverse (2f).

8. Manipulateur selon l'une quelconque des revendications 1 à 7, **caractérisé en ce qu'**il comprend un élément d'appui intermédiaire (10) du bras principal (1), dans lequel le bras principal (1) peut coulisser axialement (11), et qui peut pivoter selon un mouvement de pivotement global sphérique (8b).

9. Manipulateur selon la revendication 8, **caractérisé en ce que** le bras principal (1) peut en outre avoir, par rapport à l'élément d'appui intermédiaire (10), un mouvement de rotation globale axiale (6a).

10. Manipulateur selon l'une des revendications 8 ou 9, **caractérisé en ce que** l'élément d'appui intermédiaire est un trocart chirurgical (10) ayant des moyens de connexion à contacts glissants (10b) pour transmettre l'énergie électrique et les signaux entre le bras principal (1) et un ensemble externe d'alimentation et de traitement (200).

11. Manipulateur selon l'une quelconque des revendications 1 à 10, **caractérisé en ce que** la structure distale commandée (3) comprend un support distal (14) articulé en bout de bras principal (1), pouvant osciller de part et d'autre de l'axe longitudinal (I-I) du bras principal (1) selon des mouvements de pivotement relatif à deux degrés de liberté à axes transversaux concourants, et portant une broche rotative porte-outil (15) apte à tourner en rotation propre axiale dans le support distal (14).

12. Manipulateur selon la revendication 11, **caractérisé en ce que** les capteurs de sollicitation (2c), les moyens moteurs (5) et les moyens de transmission mécanique (6) sont agencés de façon que :
- une sollicitation de pivotement relatif centré (7a) appliquée sur la structure proximale de manipulation (2) produit un pivotement relatif (7b) similaire du support distal (14) de la structure distale commandée (3),
- une sollicitation de rotation propre axiale (9a) appliquée sur la structure proximale de manipulation (2) produit une rotation relative axiale (9b) similaire de la broche rotative porte-outil (15).

13. Manipulateur selon l'une quelconque des revendications 1 à 12, **caractérisé en ce que** la structure distale commandée (3) comprend :
- à l'extrémité distale (1b) du bras principal (1), un élément d'articulation femelle (12) à cavité hémisphérique distale (13),
- un élément d'articulation mâle (14) en forme de collerette hémisphérique creuse, ayant une surface externe hémisphérique (14a), et ayant un évidement intérieur (14b) largement ouvert vers sa base (14c),
- l'élément d'articulation mâle (14) étant engagé dans la cavité hémisphérique distale (13) de l'élément d'articulation femelle (12), avec son évidement intérieur (14b) orienté vers la cavité hémisphérique distale (13),
- une pluralité de fils de commande (23a, 23b) s'étendant dans le bras principal (1), couplés mécaniquement aux moyens moteurs (5), et engagés en périphérie de la surface externe hémisphérique (14a) de l'élément d'articulation mâle (14) pour en commander le pivotement par traction sur les fils de commande (23a, 23b),
- un arbre de sortie (15), monté à rotation dans un palier radial (16) de l'élément d'articulation mâle (14), et portant un outil (105a) ou un porte-outil (105b),
- un arbre d'entrée (17), monté à rotation dans un palier axial (18) de l'élément d'articulation femelle (12), et engagé longitudinalement dans le bras principal (1),
- une transmission homocinétique (19) qui relie l'arbre d'entrée (17) à l'arbre de sortie (15) en autorisant les mouvements de pivotement transversal de l'élément d'articulation mâle (14) dans l'élément d'articulation femelle (12).

14. Manipulateur selon la revendication 13, **caractérisé en ce que** les fils de commande (23a, 23b) traversent des passages longitudinaux de guidage périphériques (24a, 24b) à leur entrée dans l'élément d'articulation femelle (12).

## Claims

1. Manipulator, comprising:
- a main arm (1) having a proximal end (1a) and a distal end (1b),
- a proximal manipulator structure (2) carried by the proximal end (1a) of the main arm (1), to which it is connected by a connecting structure (2e, 2f, 2g), having at least two opposite contact areas (2a, 2b) conformed to have two opposite parts of a hand of an operator (100) bear on them, and including stress sensors (2c) adapted to generate movement instructions as a function of the stresses applied by the hand of the operator (100),
- a distal controlled structure (3), carried by the distal end (1b) of the main arm (1) and mobile relative to said distal end (1b) with at least two degrees of freedom in transverse relative pivoting (7b),
- drive means (5) supplied with power by a power supply (200) and adapted to generate movements as a function of movement instructions received from the proximal manipulator structure (2),
- mechanical transmission means (6) accommodated in the main arm (1), mechanically coupled to the drive means (5) and adapted to transmit movements of the drive means (5) to the distal controlled structure (3) to generate the movements of the distal controlled structure (3) as a function of the movement instructions coming from the proximal manipulator structure (2),
**characterized in that**:
- the distal controlled structure (3) can move relative to said distal end (1b) with one degree of freedom in axial rotation on itself (9b),
- the connecting structure (2e, 2f, 2g) is disposed in an intermediate area between the opposite contact areas (2a, 2b) of the proximal manipulator structure (2),
- stress sensors (2c) are disposed in said connecting structure (2e, 2f, 2g) and adapted to generate the instructions for movement of the distal controlled structure (3) relative to the main arm (1) with at least two degrees of freedom of movement as a function of the stresses detected in said connecting structure (2e, 2f, 2g).

2. Manipulator according to claim 1, **characterized in that** the connecting structure (2e, 2f, 2g) includes an articulation and the sensors (2c) are displacement sensors sensitive to the relative displacement of the main arm (1) and the proximal manipulator structure (2) on either side of the articulation.

3. Manipulator according to claim 1, **characterized in that** the connecting structure (2e, 2f, 2g) is an elastically deformable structure and the sensors are strain gauges sensitive to deformation of the connecting structure (2e, 2f, 2g).

4. Manipulator according to any of claims 1 to 3, **characterized in that**:
- the proximal manipulator structure (2) is offset radially away from the longitudinal axis (I-I) of the main arm (1),
- the opposite contact areas (2a, 2b) are, in a median position, aligned in a direction (II-II) at an angle of approximately 45° to the longitudinal axis (I-I) of the main arm (1),
- the stress sensors (2c) disposed in the connecting structure (2e, 2f, 2g) are adapted to generate the instructions for movement of the distal controlled structure (3) in accordance with the two degrees of freedom in relative transverse pivoting (7b) and the one degree of freedom in axial rotation on itself (9b).

5. Manipulator according to any of claims 1 to 3, **characterized in that**:
- the connecting structure (2e, 2f, 2g) is centered on the longitudinal axis (I-I) of the main arm (1),
- the stress sensors (2c) disposed in the connecting structure (2e, 2f, 2g) are adapted to generate the instructions for movement of the distal controlled structure (3) in accordance with the two degrees of freedom of movement in relative transverse pivoting (7b),
- the movements of axial rotation on itself of the distal controlled structure (3) are commanded by the movement instructions generated by an additional stress sensor (2j) carried by the proximal manipulator structure (2) and adapted to be actuated by a finger of the user acting on the proximal manipulator structure (2).

6. Manipulator according to any of claims 1 to 5, **characterized in that** the proximal manipulator structure (2) includes a convex external surface two diametrically opposed portions of which constitute said opposite contact areas (2a, 2b).

7. Manipulator according to any of claims 1 to 5, **characterized in that** the proximal manipulator structure (2) includes two coplanar rings constituting said opposite contact areas (2a, 2b) connected by a crosspiece (2f).

8. Manipulator according to any of claims 1 to 7, **characterized in that** it includes an intermediate bearing member (10) for the main arm (1) in which the main arm (1) can slide axially (11) and which can pivot with a spherical overall pivoting movement (8b).

9. Manipulator according to claim 8, **characterized in that** the main arm (1) may further have a movement of overall axial rotation (6a) relative to the intermediate bearing member (10).

10. Manipulator according to either of claims 8 or 9, **characterized in that** the intermediate bearing member is a surgical trocar (10) having contact means with sliding contacts (10b) for transmitting electrical power and signals between the main arm (1) and an external power supply and processing system (200).

11. Manipulator according to any one of claims 1 to 10, **characterized in that** the distal controlled structure (3) comprises a distal support (14) articulated to the end of the main arm (1), adapted to oscillate on either side of the longitudinal axis (I-I) of the main arm (1) in relative pivoting movement with two degrees of freedom about intersecting transverse axes and carrying a tool-holder rotary shaft (15) adapted to turn in axial rotation on itself on the distal support (14).

12. Manipulator according to claim 11, **characterized in that** the stress sensors (2c), the drive means (5) and the mechanical transmission means (6) are adapted so that:
- a stress for centered relative pivoting (7a) applied to the proximal manipulator structure (2) produces similar relative pivoting (7b) of the distal support (14) of the distal controlled structure (3),
- a stress for axial rotation on itself (9a) applied to the proximal manipulator structure (2) produces similar relative axial rotation (9b) of the tool-holder rotary shaft (15).

13. Manipulator according to any of claims 1 to 12, **characterized in that** the distal controlled structure (3) includes:
- at the distal end (1b) of the main arm (1), a female articulation member (12) with a hemispherical distal cavity (13),
- a male articulation member (14) in the form of a hollow hemispherical flange having a hemispherical external surface (14a) and an interior void (14b) wide open toward its base (14c),
- the male articulation member (14) being engaged in the distal hemispherical cavity (13) of the female articulation member (12) with its interior void (14b) oriented toward the distal hemispherical cavity (13),
- a plurality of control lines (23a, 23b) extending in the main arm (1), mechanically coupled to the drive means (5) and engaged at the periphery of the hemispherical external surface (14a) of the male articulation member (14) to command pivoting thereof by traction on the control lines (23a, 23b),
- an output shaft (15) rotatably mounted on a radial bearing (16) of the male articulation member (14) and carrying a tool (105a) or a tool-holder (105b),
- an input shaft (17) mounted to rotate in an axial bearing (18) of the female articulation member (12) and engaged longitudinally in the main arm (1),
- a homokinetic transmission (19) that connects the input shaft (17) to the output shaft (15), allowing movements of transverse pivoting of the male articulation member (14) in the female articulation member (12).

14. Manipulator according to claim 13, **characterized in that** the control lines (23a, 23b) pass through peripheral longitudinal guide passages (24a, 24b) on entry into the female articulation member (12).

## Patentansprüche

1. Manipulator, mit:
- einen Hauptarm (1), der ein proximales Ende (1a) und ein distales Ende (1b) aufweist,
- einer proximalen Manipulationsstruktur (2), die von dem proximalen Ende (1a) des Hauptarmes (1) getragen ist, an die sich eine Verbindungsstruktur (2e, 2f, 2g) anschliesst, die mindestens zwei entgegengesetzte Kontaktzonen (2a, 2b) aufweist, die angepasst sind, um zwei entgegengesetzte Teile einer Hand eines Operateurs (100) aufzunehmen, und die Beanspruchungssensoren (2c) aufweisen, die ausgebildet sind, um Bewegungsbefehle in Abhängigkeit von der Beanspruchung der Hand des Operateurs (100) zu erzeugen,
- einer distalen gesteuerten Struktur (3), die von dem distalen Ende (1b) des Hauptarmes (1) getragen ist und in Bezug auf das genannte distale Ende (1b) längs mindestens zwei Freiheitsgraden einer relativen transversalen Schwenkung (7b) beweglich ist,
- motorischen Mitteln (5), die von einer Energiequelle (200) gespeist werden und ausgebildet sind, um Bewegungen in Abhängigkeit von Bewegungsinstruktionen auszuführen, die von der proximalen Manipulationsstruktur (2) empfangen werden,
- mechanischen Transmissionsmitteln (6), die in dem Hauptarm (1) angeordnet sind, mechanisch mit den motorischen Mitteln (5) gekoppelt sind und dazu ausgebildet sind, Bewegungen der motorischen Mittel (5) auf die distale gesteuerte Struktur (3) zu übertragen, um Bewegungen der gesteuerten distalen Struktur (3) zu erzeugen, in Abhängigkeit von Bewegungsinstruktionen der proximalen Manipulationsstruktur (2),
**dadurch gekennzeichnet, dass**:
- die distal gesteuerte Struktur (3) in Bezug auf das distale Ende (1b) gemäss einem Freiheitsgrad der Rotation um ihre eigene Achse (9b) beweglich ist;
- die Verbindungsstruktur (2e, 2f, 2g) in einer Zwischenzone zwischen den entgegengesetzten Kontaktzonen (2a, 2b) der proximalen Manipulationsstruktur (2) angeordnet ist,
- die Beanspruchungssensoren (2c) in der genannten Verbindungsstruktur (2e, 2f, 2g) angeordnet sind und dazu ausgebildet sind, Bewegungsinstruktionen der distalen gesteuerten Struktur (3) in Bezug auf den Hauptarm (1) gemäss mindestens zwei Freiheitsgraden der Bewegung zu erzeugen, in Abhängigkeit von erfassten Beanspruchungen in der genannten Verbindungsstruktur (2e, 2f, 2g).

2. Manipulator nach Anspruch 1, **dadurch gekennzeichnet, dass** die Verbindungsstruktur (2e, 2f, 2g) ein Gelenk aufweist und die Sensoren (2c) Bewegungssensoren sind, die auf relative Bewegung zwischen dem Hauptarm (1) und der proximalen Manipulationsstruktur (2) beidseitig des Gelenkes ansprechen.

3. Manipulator nach Anspruch 1, **dadurch gekennzeichnet, dass** die Verbindungsstruktur (2e, 2f, 2g) eine elastisch verformbare Struktur ist und die Sensoren Dehnungsmessstreifen sind, die auf eine Verformung der Verbindungsstruktur (2e, 2f, 2g) ansprechen.

4. Manipulator nach irgendeinem der Ansprüche 1 bis 3, **dadurch gekennzeichnet, dass**:
- die proximale Manipulationsstruktur (2) radial in Bezug auf die Längsachse (I-I) des Hauptarmes (1) versetzt ist,
- die entgegengesetzten Kontaktzonen (2a, 2b) in mittlerer Position in einer Richtung (II-II) ausgerichtet sind, die mit der Längsachse (I-I) des Hauptarmes (1) einen Winkel von ungefähr 45° bildet,
- die Beanspruchungssensoren (2c), die in der Verbindungsstruktur (2e, 2f, 2g) angeordnet sind, dazu ausgebildet sind, Bewegungsinstruktionen der distalen gesteuerten Struktur (3) gemäss den zwei Freiheitsgraden der relativen transversalen Verschwenkung (7b) und gemäss dem Freiheitsgrad der Drehung um ihre eigene Achse (9b) zu erzeugen.

5. Manipulator nach irgendeinem der Ansprüche 1 bis 3, **dadurch gekennzeichnet, dass**:
- die Verbindungsstruktur (2e, 2f, 2g) auf der Längsachse (I-I) des Hauptarmes (1) zentriert ist,
- die Beanspruchungssensoren (2c), die in der Verbindungsstruktur (2e, 2f, 2g) angeordnet sind, ausgebildet sind, um Bewegungsinstruktionen der distalen gesteuerten Struktur (3) längs den zwei Freiheitsgraden der relativen transversalen Verschwenkung (7b) zu erzeugen,
- die Rotationsbewegungen um die eigene Achse der distalen gesteuerten Struktur (3) von den Bewegungsinstruktionen gesteuert werden, die von einem zusätzlichen Beanspruchungssensor (2j) erzeugt werden, der von der proximalen Manipulationsstruktur (2) getragen ist und von einem Finger der Hand des Benutzers, der die proximale Manipulationsstruktur (2) bedient, betätigbar ist.

6. Manipulator nach irgendeinem der Ansprüche 1 bis 5, **dadurch gekennzeichnet, dass** die proximale Manipulationsstruktur (2) eine äussere konvexe Oberfläche hat, deren diametral gegenüberliegende Teile die genannten gegenüberliegenden Kontaktzonen (2a, 2b) bilden.

7. Manipulator nach irgendeinem der Ansprüche 1 bis 5, **dadurch gekennzeichnet, dass** die proximale Manipulationsstruktur (2) zwei koplanare Ringe aufweist, die die genannten gegenüberliegenden Kontaktzonen (2a, 2b) bilden und die mit einer Traverse (2f) verbunden sind.

8. Manipulator nach irgendeinem der Ansprüche 1 bis 7, **dadurch gekennzeichnet, dass** er ein zwischenliegendes Anschlagelement (10) des Hauptarmes (1) aufweist, in welchem der Hauptarm (1) axial (11) gleiten kann und das gemäss einer generell sphärischen Schwenkbewegung (8b) schwenken kann.

9. Manipulator nach Anspruch 8, **dadurch gekennzeichnet, dass** der Hauptarm (1) zusätzlich in Bezug auf das zwischenliegende Anschlagelement (10) eine generell axiale (6a) Drehbewegung haben kann.

10. Manipulator nach einem der Ansprüche 8 oder 9, **dadurch gekennzeichnet, dass** das zwischenliegende Anschlagelement ein chirurgischer Trokar (10) ist, der Verbindungsmittel mit gleitenden Kontakten (10b) hat, um elektrische Energie und Signale zwischen dem Hauptarm (1) und einer externen Versorgungs- und Behandlungseinrichtung (200) zu übertragen.

11. Manipulator nach irgendeinem der Ansprüche 1 bis 10, **dadurch gekennzeichnet, dass** die distale gesteuerte Struktur (3) ein distales Lager (14) aufweist, das am Ende des Hauptarmes (1) angelenkt ist, um in Bezug auf beide Seiten der Längsachse (I-I) des Hauptarmes (1) zu oszillieren gemäss relativen Schwenkbewegungen mit zwei Freiheitsgraden bezüglich sich schneidender quer verlaufender Achsen und eine drehbare Welle (15) eines Werkzeugträgers trägt, die ausgebildet ist, sich um ihre eigene Achse in dem distalen Lager (14) zu drehen.

12. Manipulator nach Anspruch 11, **dadurch gekennzeichnet, dass** die Beanspruchungssensoren (2c), die motorischen Mittel (5) und die Mittel (6) zur mechanischen Transmission derart ausgebildet sind, dass:
- eine Beanspruchung für ein zentriertes relatives Schwenken (7a), die auf die proximale Manipulatorstruktur (2) ausgeübt wird, ein ähnliches relatives Schwenken (7b) des distalen Lagers (14) der distalen gesteuerten Struktur (3) bewirkt, und
- eine Beanspruchung für eine axiale Drehung um sich selbst (9a), die auf die proximale Manipulatorstruktur (2) ausgeübt wird, eine ähnliche relative axiale Drehung (9b) der drehbaren Welle (15) des Werkzeugträgers bewirkt.

13. Manipulator nach irgeneinem der Ansprüche 1 bis 12, **dadurch gekennzeichnet, dass** die distale gesteuerte Struktur (3) enthält:
- am distalen Ende (1b) des Hauptarmes (1) ein weibliches Artikulationselement (12) mit distalem hemispärischem Hohlraum (13),
- ein männliches Artikulationselement (14) in Form eines hohlen hemisphärischen Schaftes, der eine hemisphärische äussere Oberfläche (14a) hat und ein hohles Inneres (14b), das in Richtung zu seiner Basis (14c) weit offen ist,
- wobei das männliche Artikulationselement (14) in den hemisphärischen distalen Hohlraum (13) des weiblichen Artikulationselementes (12) eingreift, wobei sein hohles Inneres (14b) in Richtung zu dem distalen hemisphärischen Hohlraum (13) ausgerichtet ist,
- eine Vielzahl von Steuerdrähten (23a, 23b), die sich in dem Hauptarm (1) erstrecken, mechanisch mit den motorischen Mitteln (5) gekoppelt sind und mit dem Umfang der äußeren hemisphärischen Oberfläche (14a) des männlichen Artikulationselementes (14) in Eingriff stehen, um damit das Schwenken mittels eines Zuges an den Steuerdrähten (23a, 23b) zu bewirken,
- einen Ausgangszweig (15), der drehbar an einem radialen Lager (16) des weiblichen Artikulationselementes (14) angebracht ist und ein Werkzeug (105a) oder einen Werkzeugsträger (105b) trägt,
- einen Eingangszweig (17), der drehbar in einem Axiallager (18) des weiblichen Artikulationselementes (12) angebracht ist und longitudinal in den Hauptarm (1) eingreift,
- eine homokinetische Transmission (19), die den Eingangszweig (17) mit dem Ausgangszweig (15) verbindet, indem sie Bewegungen eines transversen Verschwenkens des männlichen Artikulationselementes (14) in dem weiblichen Artikulationselement (12) erlaubt.

14. Manipulator nach Anspruch 13, **dadurch gekennzeichnet, dass** die Steuerdrähte (23a, 23b) durch periphere längsverlaufende Führungspassagen (24a, 24b) an ihrem Eingang zu dem weiblichen Artikulationselement (12) hindurch verlaufen.
